Europäisches Patentamt

European Patent Office

Office européen des brevets

(19)

(11) Publication number: **0 066 957**
**B1**

(12) **EUROPEAN PATENT SPECIFICATION**

(45) Date of publication of patent specification: **05.12.84**

(51) Int. Cl.³: **C 12 P 19/06**

(21) Application number: **82302391.6**

(22) Date of filing: **11.05.82**

(54) **Inoculation procedure for xanthan gum production.**

(30) Priority: **22.05.81 GB 8115855**

(43) Date of publication of application:
**15.12.82 Bulletin 82/50**

(45) Publication of the grant of the patent:
**05.12.84 Bulletin 84/49**

(84) Designated Contracting States:
**DE FR GB NL**

(56) References cited:
**FR-A-2 409 309**
**FR-A-2 414 555**
**US-A-3 594 280**
**US-A-4 301 247**

(73) Proprietor: **Kelco Biospecialties Limited**
**22 Henrietta Street**
**London WC2E 8NB (GB)**

(72) Inventor: **Rodney, Jarman Trevor**
**18 Moss Drive**
**Haslingfield Cambridgeshire (GB)**

(74) Representative: **Ablewhite, Alan James et al**
**MARKS & CLERK 57/60 Lincoln's Inn Fields**
**London WC2A 3LS (GB)**

Courier Press, Leamington Spa, England.

## Description

The present invention relates to the production of xanthan gum.

Xanthan gum is a polysaccharide elaborated by many species of the genus *Xanthamonas*. The gum has interesting rheological properties, and is used particularly as a thickener in oil drilling and foodstuffs.

Industrial production of xanthan gum is generally by a batch process, in which a culture of a polysaccharide-producing strain of *Xanthamonas* is inoculated into a nutrient medium in a fermentation vessel and allowed to ferment for up to several days. Thereafter the gum is usually isolated from the fermented broth by addition of an organic solvent to precipitate the gum.

An alternative to batch production production is continuous production, in which after inoculation and initial growth, fresh medium is continuously added to the fermentation vessel and a corresponding amount of fermented broth is continuously withdrawn. The advantages of continuous xanthan gum production, especially regular production, ease of control and smaller fermentation vessel, have been recognized for many years. However, for various reasons, particularly practical difficulties in maintaining extended, productive fermentation, batch production still predominates.

An essential part of a process for production of xanthan gum is the inoculation step, in which a culture of the productive strain is added to the nutrient medium as a prelude to the onset of productive fermentation. Typically the nutrient medium used for production has a volume of 10 to 20 times the volume of the inoculum, corresponding to a dilution of 10 to 5%. Greater dilutions are avoided because of the strong possibility that the inoculating strain will not commence active growth in the production medium.

Unexpectedly, as part of the discovery of the process described in our EP—A—0 066 377, we have found that under certain conditions it is feasible to employ dilutions of less than 1%. The use of inoculum dilutions of as low as "less than 0.5 to 1 volume per 1000 volumes" is reported in US 3594 280 in batch culture in a medium with an undefined trace element content. However, we have found that careful control of the medium gives very good results, especially in continuous fermentations.

According to the present invention we provide as method of inoculating a production medium with a polysaccharide-producting strain of *Xanthomonas* for subsequent culturing to produce a polysaccharide xanthan gum, wherein an inoculum is mixed at dilution of 0.5% or less with a production medium containing iron, manganese, zinc, copper, cobalt and boron in controlled low levels.

We have found that inocula can be successfully employed to inoculate production media on a basis of 1 part by volume of inoculum to at least 200 parts by volume of production medium, provided that the production medium contains low levels of the trace elements. Without being bound by any theory, it appears that the customary higher levels of the specified trace elements have an inhibitory effect at high inoculation dilutions.

More particularly, in accordance with the present invention the inoculum is used with a production medium which contains total levels of no more than 10 ppm of iron, no more than 3 ppm of manganese and no more than 3 ppm of zinc ('ppm' in this specification being parts by weight of the element per million parts by volume of the medium).

The volume ratio of inoculum: production medium is preferably 1:500 to 1:10,000, more preferably 1:1000 to 1:5000. By the use of such volume ratios there is the possibility of a substantial saving in manufacturing plant and a concomitant reduction in processing of the inoculum.

Consider, for example, a plant wherein the main fermenter vessel has a liquid volume of 10 m³. Starting with a culture of volume 1 litre, and assuming a conventional dilution at each stage of about 10%, it is necessary to go through the sequence:

$$\text{1 litre} \rightarrow \text{10 litre} \rightarrow \text{100 litre} \rightarrow \text{1 cubic metre} \rightarrow \text{10 cubic metre}$$

In contrast, with the present invention, the 1 litre can be used at 10% dilution to produce 10 litre of inoculum which at 0.1% dilution enables direct inoculation of 10 m³ of production medium in the main fermentation vessel.

Thus, for the example under consideration, the vessels conventionally needed to hold the 100 litre and the 1 m³ stages as part of the inoculum preparation can now be dispensed with. Not only are there capital savings, but also there is less risk of contamination because there are fewer transfers and also there is generally less work involved.

It will be appreciated that, with the inoculation procedure of the invention, there is normally a longer growing-up period than before. Many hours are required before the bacteria present at a given concentration in 1 volume of inoculum will grow up to the same concentration in 200 or more volumes of production medium. The grow-up time is typically 10 to 100 hours. For this reason, the present invention is best suited to continuous fermentation processes.

Measures can be taken to minimise the grow-up time. A particularly preferred measure is to use substantially the same medium for at least the final stage of the inoculum preparation as is used for the production medium. Thus, for the example considered above where 10 m³ of medium is to be

2

inoculated, the 1 litre of initial culture should be added to a sample of 9 litre of production medium, thereby giving the 10 litre inoculum grown in the same medium. This measure serves to pre-adapt the bacterium to growth in the production medium.

Another preferred measure is for the inoculation to be effected when the bacterium is actively growing, rather than in any dormant phase. It is also preferred to minimise grow-up time by controlling the oxygen supply: the air flow and mixing conditions are preferably adjusted to keep the dissolved oxygen tension in the inoculated production medium at above the levels which cause oxygen limitation and below the levels which cause oxygen toxicity.

Finally, it is also preferred to minimise grow-up times by use of a medium containing further trace elements and auxiliary components at controlled levels.

The production medium will contain assimilable sources of carbon and nitrogen. Glucose and ammonium phosphate respectively, are suitable sources of these elements, but preferably these are supplemented with an auxiliary component which is a complex naturally-derived mixture containing organic nitrogen compounds. In particular, yeast extract should be added at 0 to 5 g/l, suitably 0 to 1 g/l. Yeast extract or similar auxiliary components appear to help minimise grow-up times, particularly under the 'stress' conditions encountered in the dilution which is employed in the present invention.

Moreover, it is preferred that the production medium contains controlled, low total levels of the trace elements copper, cobalt and boron. The copper, cobalt and boron are each preferably at 2 ppm or less, better still 1 ppm or less.

Thus, for preferance, the production medium contains the following total levels of the trace elements specified in Table 1:

TABLE 1

| Trace element | Preferred level (ppm) | Most preferred level (ppm) |
|---|---|---|
| Fe | 0.1 to 10 | 0.5 to 5 |
| Mn | 0.01 to 3 | 0.1 to 1 |
| Zn | 0.01 to 3 | 0.1 to 0.5 |
| Cu | 0.001 to 2 | 0.01 to 0.1 |
| Co | 0.001 to 2 | 0.01 to 0.1 |
| B | 0 to 2 | 0.005 to 0.05 |

More generally, the present invention usually employs iron at greater levels than manganese, with the iron: manganese ppm ratio preferably being from 40:1 to 2:1.

In preparing a fermentation medium for use in the present process, the various trace elements are suitably added as water-soluble compounds, especially inorganic salts such as $FeCl_2 \cdot 6H_2O$ of $FeSO_4 \cdot 7H_2O$ for iron; $MnCl_2 \cdot 6H_2O$ or $MnSO_4 \cdot 4H_2O$ for manganese; $ZnSO_4 \cdot 7H_2O$ for zinc; $CuCl_2 \cdot 2H_2O$ or $CuSO_4 \cdot 5H_2O$ for copper; $CoCl_2 \cdot 6H_2O$ or $CoCO_4 \cdot 7H_2O$ for cobalt; and $H_3BO_3$ for boron.

In adding the trace elements, it is necessary to make due allowance for the amounts present in other components. It is greatly preferred to employ demineralized water prepared for example by passing tap water through an ion exchanger. Equally, it is greatly preferred to analyse the medium after it has been made up, in order to confirm that the controlled, low levels of trace elements have not been exceeded.

Other elements will usually be present in the production medium, especially potassium, phosphorus and sulphur. These elements can be added at conventional levels since it is not apparent that any advantage stems from altering the levels. The medium preferably has a pH of 6 to 8, and is preferably held at 29 to 32°C for the growing up.

The present invention is suited for use with any polysaccharide-producing strain of *Xanthomonas*, but for preference a strain of *X. campestris* is used.

Once the growing up of the bacterium has been completed to the desired level in the intended volume of medium, the production fermentation can be commenced. With a continuous process, extra medium is continuously added and fermented broth is continuously drawn off. For preference the dilution rate is 0.01 to 0.12 $h^{-1}$.

The fermented broth can be further purified if desired so as to give a solid xanthan gum product. In a preferred process, the broth is heat-treated at 100 to 140°C for 1 to 10 minutes, more preferably 125 to 130°C for 1 to 5 minutes, and then mixed with IPA causing precipitation of the gum which can be filtered off or otherwise separated.

The present invention is illustrated by the following examples.

Examples 1 to 4

Small scale fermentations

Working stocks of *X. campestris* (derived from ATCC 13951) were maintained on TGY agar slopes for a maximum of 14 days. The TGY medium had the composition shown in Table 2:

TABLE 2

| Tryptone | 5 gl$^{-1}$ |
|---|---|
| Glucose | 2 gl$^{-1}$ |
| Yeast Extract | 5 gl$^{-1}$ |
| Agar | 20 gl$^{-1}$ |

Fermenter inocula for examples 1 and 3 were prepared by suspending the growth on a TGY slope in 10 ml Ringers solutions (1/4 strength) and using 2 ml to inoculate 100 ml MYGP in 500 ml shake flasks. The MYGP medium had the composition shown in Table 3:

TABLE 3

| Malt Extract | 3 gl$^{-1}$ |
|---|---|
| Yeast Extract | 3 gl$^{-1}$ |
| Peptone | 5 gl$^{-1}$ |
| Glucose | 10 gl$^{-1}$ |

After 24 h incubation at 30°C on a gyrotatory shaker the MYGP broth was used for inoculation. For examples 2 and 4 a sample from a continuous culture was used as an inoculum. The continuous culture had been set in operation in the way described for Example 1 except that 10% inoculum level was employed.

For each of Examples 1 to 4, 2 litre scale *Xanthomonas* fermentations were started using 0.1 or 0.01% inoculum levels to inoculate media of the composition shown in Table 4 when read in conjunction with Table 5.

4

TABLE 4
Medium composition for fermenter cultures

| | Examples 1 to 4 | Example 5 |
|---|---|---|
| Dexyme 081 Glucose Syrup | 60 gl$^{-1}$ | 53 gl$^{-1}$ |
| Citric Acid | 1 gl$^{-1}$ | 1.0 gl$^{-1}$ |
| Acetic Acid | 0.45 ml | — |
| Ca(OH)$_2$ | 0.05 gl$^{-1}$ | 0.05 gl$^{-1}$ |
| Mg(OH)$_2$ | 0.0775 gl$^{-1}$ | 0.07 gl$^{-1}$ |
| MgSO$_4 \cdot$ 7H$_2$O | 0.058 gl$^{-1}$ | 0.086 gl$^{-1}$ |
| (NH$_4$)$_2$HPO$_4$ | 3.0 gl$^{-1}$ | 2.38 gl$^{-1}$ |
| K$_2$HPO$_4$ | 1.0 gl$^{-1}$ | 1.0 gl$^{-1}$ |
| MnSO$_4 \cdot$ H$_2$O | 0.83 mgl$^{-1}$ | 0.83 mgl$^{-1}$ |
| ZnSO$_4 \cdot$ 7H$_2$O | 1.42 mgl$^{-1}$ | 0.71 mgl$^{-1}$ |
| CuSO$_4 \cdot$ 5H$_2$O | 0.25 mgl$^{-1}$ | 0.25 mgl$^{-1}$ |
| CoSO$_4 \cdot$ 7H$_2$O | 0.28 mgl$^{-1}$ | 0.28 mgl$^{-1}$ |
| H$_3$BO$_4$ | 0.06 mgl$^{-1}$ | 0.06 mgl$^{-1}$ |
| FeSO$_4 \cdot$ 7H$_2$O | 35.6 mgl$^{-1}$ | 35.6 mgl$^{-1}$ |
| Yeast Extract | 0 or 0.2 gl$^{-1}$ | 0.2 gl$^{-1}$ |
| Deionized Water | to 1000 ml | to 1000 ml |

The glucose syrup was selected because of its purity, the Dexyme 081 being low in trace elements. In ppm, the syrup contained the following: Zn, less than 0.01; Cu, less than 0.2; Mn, less than 0.04; Co, less than 0.08; Fe, less than 0.18.

The yeast extract was a special low salts yeast extract, and contained in ppm: Zn, 60; Cu, 10; Mn, 15; Co, less than 5; Fe, 100.

The other reagents were of analytical grade.

TABLE 5

| Example No. | Inoculum preparation | Inoculum level (% v/v) | Yeast extract | Time to reach $E_{540}$ of 3.0 (h) |
|---|---|---|---|---|
| 1 | Shake flask; MYGP | 0.1 | No | 86 |
| 2 | Continuous culture; Production medium | 0.1 | Yes | 50 |
| 3 | Shake flask; MYGP | 0.1 | No | 90 |
| 4 | Continuous culture; Production medium | 0.01 | No | 106 |

Each culture was monitored during batch phase by measuring regularly the cell mass using turbidity measurements (adsorption at 540 nm ($E_{540}$)) and dissolved oxygen tension (DOT). To avoid oxygen limitation or oxygen toxicity, the air flow and stirring speed were adjusted to keep the DOT within the range 15—45% of air saturation. For the 0.1% inoculum level cultures this meant an initial air flow of 1.0 l min$^{-1}$ and a stirring speed of 50 rpm. For the culture inoculated at 0.01% level no stirring was necessary until 85 h, the agitation from the air flow of 1.01 min$^{-1}$ providing adequate mixing and oxygen transfer. The air flow was later turned up to 2.01 min$^{-1}$ and the stirring speed increased as the DOT dropped below 15%. Other fermentation conditions were temperature 30$\pm$0.05°C, pH 7.0$\pm$0.1 and ungassed working volume 2 litre.

For each of Examples 1 to 4, the inoculation was successful and continuous fermentation to produce xanthan gum was possible. The cultures differed in the time taken to reach the desired working biomass level before the switch from batch to continuous operation and feasible, as indicated in Table 5 for the time to reach $E_{540}$ of 3.0, corresponding to a cell mass of approximately 2 g dry weight per litre.

Example 5
Pilot scale fermentation

An inoculum was prepared in a 20 l vessel containing the medium indicated in Table 4. The medium was inoculated with 100 ml of MYGP broth from a shake flask incubated at 30°C for 48 h following inoculation from a TGY slope. Initial fermentation conditions were: temperature, 30$\pm$0.05°C; pH, 7.0$\pm$0.1; air flow, 2.0 litre min$^{-1}$; impeller speed, 50 rpm. As the DOT fell the air was turned up to 4.0 litre min$^{-1}$ and the stirring increased in steps to 350 rpm keeping the DOT between 10 and 50% of air saturation. The culture was ready for inoculating a 1000 litre culture after 58 h when $E_{540}$ was 2.3.

The inoculum was added at 0.1% to 1000 litre of the same medium.

Initial conditions were: temperature, 31$\pm$1°C; pH, 6.8$\pm$0.1; impeller speed, 35 rpm; working volume, 980 l; overpressure, 27 kPa. After 21 h the air flow and impeller speed were stepped up to keep the DOT at about 30%.

As the DOT fell the stirring was stepped up gradually to a maximum of 280 rpm and the air flow and overpressure were increased to a maximum of 1080 litre min$^{-1}$ and 100 kPa, such that DOT was maintained at 40—60% of saturation under the initial operating conditions. The procedure produced a cell doubling time of 5 h. The product concentration reached 24 gl$^{-1}$ at 46 h after inoculation, at which time the broth viscosity was about 26000 cps. Medium inflow for continuous operation was then begun, leading to production of xanthan gum broth on a continuous basis.

## Claims

1. A process of producing xanthan gum by aerobic fermentation of a nutrient medium, in which inoculation of the nutrient medium is effected at a dilution of 0.5—0.01% using an inoculum comprising an organism of the genus *Xanthomonas*, wherein the total levels of iron, manganese, and zinc in said nutrient medium are limited such that there is no more than 10 ppm iron, no more than 3 ppm zinc and the ppm ratio of iron:manganese is from 40:1 to 2:1.

2. A process of Claim 1, wherein the organism has the identifying characteristics of *X. campestris,* preferably the strain ATCC 13951, and the dilution is 0.1—0.2%.

3. A process of Claim 1, wherein the composition of the inoculum is substantially the same as the composition of the nutrient medium.

4. A process of Claim 1, wherein the inoculation is effected while the organism is actively growing.

5. A process of Claim 1, wherein the nutrient medium further comprises a complex naturally derived mixture of organic nitrogen compounds.

6. A process of Claim 1, wherein the nutrient medium comprises no more than 2 ppm copper, cobalt or boron.

7. A process of Claim 1, wherein the nutrient medium comprises:

|  | ppm |
| --- | --- |
| iron | 0.1—10 |
| manganese | 0.01—3 |
| zinc | 0.01—3 |
| copper | 0.001—2 |
| cobalt | 0.001—2 |
| boron | 0—2 |

8. A process of Claim 6, wherein the nutrient medium comprises:

|  | ppm |
| --- | --- |
| iron | 0.5—5 |
| manganese | 0.1—1 |
| zinc | 0.1—0.5 |
| copper | 0.01—0.1 |
| cobalt | 0.01—0.1 |
| boron | 0.005—0.05 |

9. A process of Claim 1 which is a continuous process with a dilution rate of 0.01 to 0.12 $h^{-1}$.

10. A process of Claim 1 comprising the further step of treating the fermented medium at 100—140°C for 1—10 minutes prior to recovery of the xanthan gum.

## Patentansprüche

1. Verfahren zur Herstellung von Xanthan-Gummi durch aerobe Fermentation eines Nährmediums, bei dem das Inokulieren des Nährmediums bei einer Verdünnung von 0,5—0,01% unter Anwendung eines Inokulums durchgeführt wird, das einen Organismus des Genus Xanthomonas enthält, wobei der Gesamtgehalt an Eisen, Mangan und Zink in dem Nährmedium derart begrenzt ist, dass nicht mehr als 10 ppm Eisen, nicht mehr als 3 ppm Zink vorhanden sind und das ppm-Verhältnis von Eisen:Mangan 40:1 bis 2:1 beträgt.

2. Verfahren nach Anspruch 1, bei dem der Organismus die Identifikationscharakteristika von X. Campestris, vorzugsweise dem Stamm ATCC 13951 hat und die Verdünnung 0,1—0,2% beträgt.

3. Verfahren nach Anspruch 1, bei dem die Zusammensetzung des Inokulums im wesentlichen die gleiche wie die Zusammensetzung des Nährmediums ist.

4. Verfahren nach Anspruch 1, bei dem das Inokulieren durchgeführt wird während der Organismus aktiv wächst.

5. Verfahren nach Anspruch 1, bei dem das Nährmedium darüberhinaus ein komplexes aus der Natur stammendes Gemisch organischer Stickstoffverbindungen enthält.

6. Verfahren nach Anspruch 1, bei dem das Nährmedium nicht mehr als 2 ppm Kupfer, Kobalt oder Bor enthält.

7. Verfahren nach Anspruch 1, bei dem das Nährmedium umfasst:

|  | ppm |
| --- | --- |
| Eisen | 0,1—10 |
| Mangan | 0,01—3 |
| Zink | 0,01—3 |
| Kupfer | 0,001—2 |
| Kobalt | 0,001—2 |
| Bor | 0—2 |

8. Verfahren nach Anspruch 6, bei dem das Nährmedium umfasst:

|  | ppm |
| --- | --- |
| Eisen | 0,5—5 |
| Mangan | 0,1—1 |
| Zink | 0,1—0,5 |
| Kupfer | 0,01—0,1 |
| Kobalt | 0,01—0,1 |
| Bor | 0,005—0,05 |

9. Verfahren nach Anspruch 1, das ein kontinuierliches Verfahren mit einer Verdünnungsrate von 0,01 bis 0,12 h$^{-1}$ ist.

10. Verfahren nach Anspruch 1, das die weitere Stufe der Behandlung des fermentierten Mediums bei 100—140°C während 1—10 Minuten vor der Gewinnung des Xanthan-Gummis umfasst.

**Revendications**

1. Procédé de production de gomme de xanthane par fermentation aérobie d'un milieu nutritif, où l'on effectue l'inoculation du milieu nutritif à une dilution de 0,5—0,01% en utilisant un inoculum comprenant un organisme du genre *Xanthomonas*, où les niveaux totaux de fer, de manganèse et de zinc dans ledit milieu nutritif sont limités de manière telle qu'il n'y ait pas plus de 10 ppm de fer, pas plus de 3 ppm de zinc et que le rapport en ppm de fer:manganèse soit compris entre 40:1 et 2:1.

2. Procédé de la revendication 1, où l'organisme a les caractéristiques d'identification de *X. campestris,* de préférence la souche ATCC 13951, et la dilution est de 0,1—0,2%.

3. Procédé de la revendication 1, où la composition de l'inoculum est essentiellement la même que la composition du milieu nutritif.

4. Procédé de la revendication 1, où l'inoculation est effectuée tandis que l'organisme est en croissance active.

5. Procédé de la revendication 1, où le milieu nutritif comprend en outre un mélange complexe d'origine naturelle de composés d'azote organiques.

6. Procédé de la revendication 1, où le milieu nutritif comprend au plus 2 ppm de cuivre, de cobalt ou de bore.

7. Procédé de la revendication 1, où le milieu nutritif comprend:

|  | ppm |
| --- | --- |
| fer | 0,1—10 |
| manganèse | 0,01—3 |
| zinc | 0,01—3 |
| cuivre | 0,001—2 |
| cobalt | 0,001—2 |
| bore | 0—2 |

8. Procédé de la revendication 6, où le milieu nutritif comprend:

|  | ppm |
| --- | --- |
| fer | 0,5—5 |
| manganèse | 0,1—1 |
| zinc | 0,1—0,5 |
| cuivre | 0,01—0,1 |
| cobalt | 0,01—0,1 |
| bore | 0,005—0,05 |

9. Procédé de la revendication 1 qui est un procédé continu avec un taux de dilution de 0,01 à 0,12 $h^{-1}$.

10. Procédé de la revendication 1 comprenant l'étape supplémentaire consistant à traiter le milieu fermenté à 100—140°C pendant 1—10 minutes avant la récupération de la gomme de xanthane.